# EUROPEAN PATENT SPECIFICATION

(11) **EP 2 961 330 B1**
(45) Date of publication and mention of the grant of the patent: **30.05.2018**
(21) Application number: 14710164.6
(22) Date of filing: 26.02.2014
(51) Int. Cl.: A61B 17/02, A61B 17/17, A61G 13/12, A61L 31/14, A61B 17/16, A61B 17/15, A61B 17/56

(54) **TOOL WITH BIOABSORBABLE MAGNESIUM ALLOY FOR INTRAOPERATIVE USE**
WERKZEUG MIT BIOABSORBIERBARER MAGNESIUMLEGIERUNG ZUR INTRAOPERATIVEN VERWENDUNG
OUTIL AVEC ALLIAGE DE MAGNÉSIUM BIOABSORBABLE POUR UTILISATION PEROPÉRATOIRE

(30) Priority: 01.03.2013 US 201361771505 P
(43) Date of publication of application: 06.01.2016
(73) Proprietor: Stryker Corporation, Kalamazoo, MI 49002 (US)
(72) Inventor: STAUNTON, Doug, Kalamazoo, MI 49009 (US)
(74) Representative: Regimbeau
(86) International application number: PCT/US2014/018631
(87) International publication number: WO 2014/134145

(56) References cited:
- DE-A1-102005 007 616
- US-A- 5 239 716
- US-A1- 2010 292 540
- US-A1- 2013 325 017

## Description

### BACKGROUND OF THE INVENTION

This invention relates generally to a surgical system comprising a stationary tool formed at least partially of a bioabsorbable material and further comprising a machining tool that may impact the bioabsorbable material during surgery.

In various surgical procedures, a machining tool, such as a bur, drill bit, or reciprocating saw moves at a high rate of speed or with a large amount of force in the vicinity of generally stationary tools, such as a retractor, jig, fixture, or cutting guide. During such procedures, a contact surface of the machining tool may make contact with a contact surface of the stationary tool. Although the contact surfaces are not generally intended to make contact, such contact has a realistic chance of occurring during a variety of procedures. This contact may result from, for example, tight working conditions, accident, or even from unintended or unanticipated movement by a robot controlling the machining tool. The contact may also be intentional, for example, guiding a reciprocating saw though a cutting slot. Even when intentional, such contact may be relatively light or heavy.

Contact between a machining tool and a stationary tool, especially when the contact occurs with high forces, may result in damage to the machining tool and/or the stationary tool. Further, such contact may cause pieces of the stationary tool, either small or large, to chip, strip, or otherwise dislodge from the stationary tool. This may be particularly dangerous if the patient being operated on has exposed incisions or wounds such that the material is capable of landing within the exposed surgical site. While large pieces of material may be easily visualized and removed, smaller pieces of material, which may be nearly imperceptible, may remain in the surgical site unnoticed, and remain even after the incision is sutured together. If the material is not biocompatible, the patient may have an adverse reaction to the material. Even if the material is biocompatible, it is preferred that the material is bioabsorbable such that material lodged in the patient's body is broken down over time. US2010292540 A1 discloses a liver sling retractor for use in laparoscopic, endoscopic, transluminal, or endoluminal surgical procedures, the retractor being constructed of absorbable material e.g. bioabsorbable magnesium alloys, polyglactin (Vicryl®), polyglycolic acid (Dexon®), or regenerated oxidized cellulose or any other bioabsorbable material, or combinations of bioabsorbable materials.

### BRIEF SUMMARY OF THE INVENTION

According to the invention, the surgical system for performing a surgical procedure includes a machining tool and a stationary tool. The machining tool has a first contact surface having a first hardness. The stationary tool has a second contact surface at least partially formed of a magnesium alloy, the second contact surface being biocompatible and bioabsorbable and having a second hardness. The first hardness is greater than the second hardness. The first contact surface may also have a toughness that is greater than a toughness of the second contact surface.

In alternative variants not forming part of the invention, the stationary tool may be entirely formed of magnesium alloy. In embodiments of the invention the second contact surface is coated with magnesium alloy. The machining tool may be selected from the group consisting of a bur, a drill bit, an end mill, and a saw. The stationary tool may be selected from the group consisting of a retractor, a jig, a fixture, and a cutting guide.

In another embodiment of the invention, a surgical system for performing a surgical procedure includes a machining tool, a stationary tool, and an insert. The machining tool has a first contact surface having a first hardness. The insert is configured to be coupled to the stationary tool, the insert having a second contact surface and being at least partially formed of a magnesium alloy. The second contact surface is biocompatible and bioabsorbable and has a second hardness. The first hardness is greater than the second hardness. The first contact surface may also have a toughness that is greater than a toughness of the second contact surface. In embodiments of the invention, the second contact surface is coated with magnesium alloy. The machining tool may be selected from the group consisting of a bur, a drill bit, an end mill, and a saw. The stationary tool may be selected from the group consisting of a retractor, a jig, a fixture, and a cutting guide.

A method of performing orthopedic surgery, the method not forming part of the claimed invention, includes creating an incision in a patient at a surgical site to at least partially expose a bone of the patient. The method also includes the step of positioning a stationary tool in the surgical site, the stationary tool having a biocompatible and bioabsorbable first contact surface and being at least partially formed of magnesium alloy. The first contact surface has a first hardness. The method further includes the step of contacting the bone with a machining tool, the machining tool having a second contact surface having a second hardness greater than the first hardness. The second contact surface may also have a toughness that is greater than a toughness of the first contact surface.

### BRIEF DESCRIPTION OF THE DRAWINGS

The invention is pointed out with particularity in the claims. The features and benefits of this invention may be better understood from the following Detailed Description taken in conjunction with the drawings.
FIG. 1 is a perspective view of a limb holder.
FIG. 2 is a perspective view of a retractor assembly that may be used with the limb holder of FIG. 1.
Fig. 3 illustrates retractors in place in an incision of a patient's knee during a surgical procedure.
Fig. 4 illustrates a proximal tibia with a resection cutting guide and vertical cutting guide attached thereto.
Fig. 5 illustrates the proximal tibia of FIG. 4 after sagittal cuts have been made.
Fig. 6 illustrates the proximal tibia of FIG. 5 after a resection has been made.

### DETAILED DESCRIPTION

In certain surgical procedures, machining tools may be likely to contact stationary tools. As used herein, machining tools generally refer to any number of tools that move at high rates of speed or that generate large amounts of force. Machining tools might include, for example, a bur, a drill bit, an end mill, saw teeth, or a reciprocating saw. Other tools that are likely to impact a stationary tool during surgery may also fall under the broad category of machining tools, whether or not such tools are automated or motorized. Stationary tools, on the other hand, refer to tools that are used in a surgical procedure with a machining tool, but which are generally stationary in comparison to the machining tool. Stationary tools might include, for example, retractors, jigs, fixtures, or cutting guides. It should be understood that a stationary tool need not be entirely stationary during a surgical procedure.

A number of factors go into the consideration of how to manufacture a machining tool to be used with a stationary tool. For example, particular materials as well as mechanical properties of those materials may be a factor to be considered in determining how to manufacture a machining tool and/or a stationary tool. Similarly, costs involved, biocompatibility, and ease of sterilization or resterilization may be considered. For purposes of illustration only, one particular system will be discussed to elicit a fuller understanding of the subject matter described herein, it being understood that the disclosure may apply to any number of surgical systems.

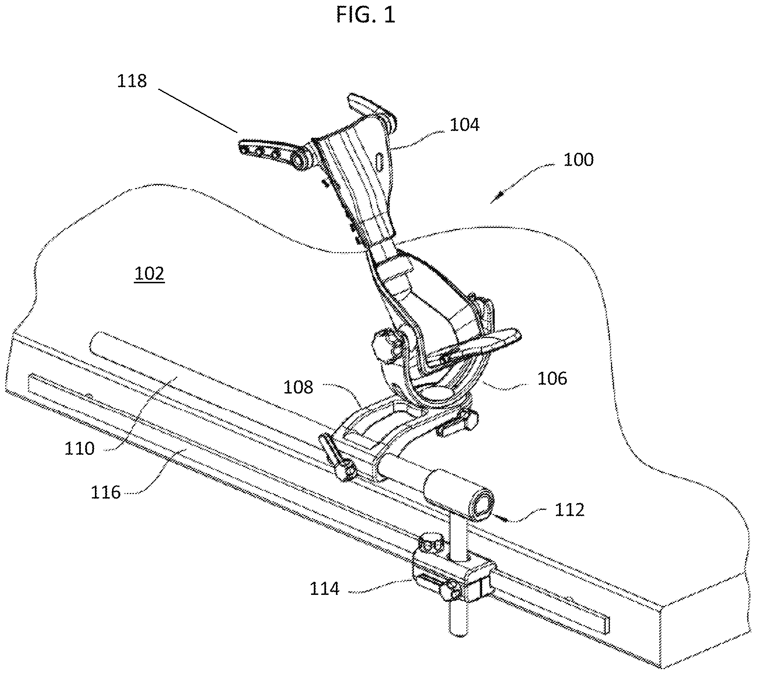

FIG. 1 illustrates an embodiment of a limb holder 100. Generally, limb holder 100 may be used to position the leg of a patient in various positions during a surgical procedure, such as a total knee arthroplasty procedure. In such a procedure, the patient lies on a table 102. The patient's foot or leg is placed within a limb support 104 and may be secured to the limb support. The limb support 104 may be coupled to a yoke 106 that is pivotable about a sled 108. The sled 108 may be translatable along a support bar 110. The support bar 110 may be coupled to a pylon 112 which is secured to a clamp 114. The clamp 114 may be clamped to a bar 116, the bar being fixed to the table 102. This combination of elements allows for a patient's foot, leg, and/or knee to be positioned in a variety of different ways to facilitate the surgical procedure. This and other limb positioning systems are more fully described in U.S. Patent Publication No. 2013/0019883.

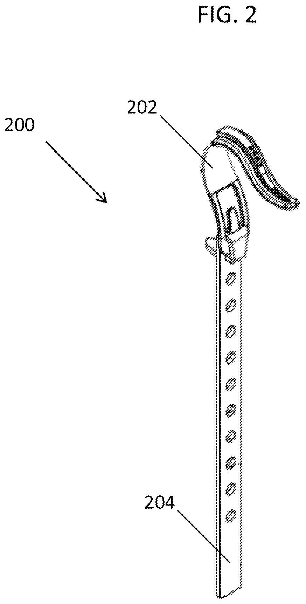

Still referring to FIG. 1, the limb support 104 may include one or more support wings 118 configured to secure one or more of a variety of attachments to the limb support. For example, a stationary tool, such as a retractor 202 of the retractor assembly 200 illustrated in FIG. 2, may be attached to a support wing 118. The retractor assembly 200 may include a retractor 202 and a strap 204 coupled to the retractor. The strap 204 may include apertures to connect to pins or fasteners of the support wing 118 in a variety of positions.

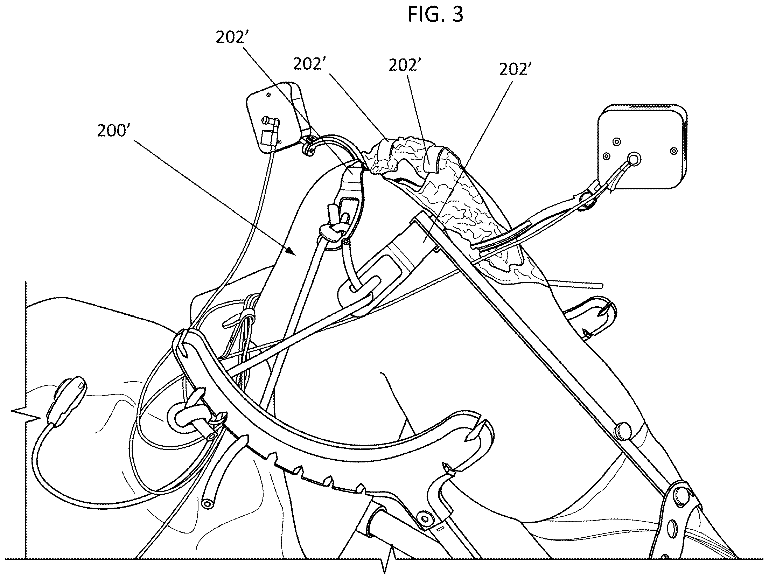

FIG. 3 illustrates a patient's knee during a surgical procedure using a system similar to the limb holder 100 described above. During an operation, such as a knee surgery, the patient's leg is secured in a limb support (not illustrated in FIG. 3) and retractor assemblies 200' are coupled to the limb support. Once the appropriate incision (s) has been made in the knee, retractors 202' are inserted into the incision to position the soft tissue and to hold the surgical site open for the surgeon. Generally, two or more retractors 202' would be used on opposite sides of the incision. As the surgery proceeds, the surgeon may use a machining tool on the bone. For example, the surgeon may use a drill bit to drill a hole into the bone, or may use a reciprocating saw to resect a surface of the bone. These machining tools are illustrative only, as any number of machining tools may be used. With one or more retractors 202' in place, there is a possibility that the machining tool may make contact with the retractor.

As described above, the contact of the machining tool with the retractor 202' (or other stationary tool) may be disfavored for a number of reasons. Particularly, this contact may deform the machining tool, for example dulling a blade or stripping a drill bit. Such deformation could lead to clinically significant inaccuracies during further use of the machining tool. If the deformation is great enough, the operation may need to be interrupted so that the machining tool can be replaced before finishing the procedure. The contact may also deform the retractor 202' or other stationary tool. For example, material of the retractor 202' may be chipped off, or the retractor may even break. Even if the retractor 202' does not break from such contact, it may be weakened. It may be unsafe to use a weakened retractor 202' because it may break from further contact or even from further normal use. This inserts a degree of uncertainty into the choice of how many times a retractor 202' or other stationary tool may be reused (after resterilization) before it is discarded. Even further, as can be seen in FIG. 3, if the retractor 202' is contacted by a machining tool, material is likely to enter the patient's body though the surgical field. If the material is not biocompatible and quickly absorbable by the patient's body, adverse reactions may occur at the surgical site. Even if the material is biocompatible, it is preferable that the material is quickly absorbable such that the body breaks down the foreign material in the body.

In order to overcome at least some of the disadvantages of contact between the machining tool and the stationary tool, such as the retractor 202', materials for the stationary tool should be carefully chosen. For example, it would be preferable that the stationary tool has a lower hardness compared to the machining tool. Hardness may be conceptualized by one way in which hardness is tested - the scratch test. If the machining tool is moved across the stationary tool and the stationary tool becomes scratched, the machining tool has a greater hardness than the stationary tool. On the contrary, if the stationary tool scratches the machining tool, the stationary tool has a greater hardness than the machining tool. Preferably, the hardness of the stationary tool is significantly less than the hardness of the machining tool, such that contact between the two will have a less damaging effect on the machining tool compared to the stationary tool. This may be beneficial as machining tools may be more expensive than stationary tools, and particularly because machining tools generally need to retain their machining features. For example, if a saw blade becomes deformed, it is likely to become dull, making it less effective. Similarly, if a drill bit becomes deformed from contact with a stationary tool, it is likely to become dulled or stripped, reducing the effectiveness of the drill bit.

Another material property that may be considered is the modulus of elasticity of the stationary tool. Often, the harder a material, the higher its modulus. A higher modulus material used in a given design will yield a stiffer part. Thus, a material with lower hardness may be more flexible with a given design than a material with greater hardness. While a low hardness is preferred for the stationary tool relative to the machining tool, it may be important to maintain a certain level of stiffness such that the stationary tool is not overly flexible. This may be particularly true for retractors, as a main function of retractors is to keep tissue in place. A highly flexible retractor may perform poorly compared to a more rigid retractor.

Similarly, the toughness of the material used for the stationary tools and machining tools may be a factor to be considered. Toughness generally refers to the energy taken up by the material before it fractures or cleaves. In other words, toughness relates to crack propagation and energy dissipation in a material. A lower toughness may be preferred for the stationary tool. A lower toughness will generally result in a smaller likelihood that, if contacted, a large piece or chunk of the stationary tool will break off. Although hardness and toughness may often track together (e.g. harder materials may generally be tougher), these properties may not always track each other with fidelity. However, if toughness of the stationary tool is too low, it may become too likely to fracture. Therefore, the toughness of the stationary tool is preferably lower relative to the toughness of the machining tool, while remaining tough enough to resist fracturing too easily from normal use.

As described above, biocompatibility as well as bioabsorbability may also be important factors in determining a material for use in stationary tools. Biocompatibility generally refers to whether or not a material irritates the body. Such irritation may be in the form of inflammation or something more serious such as the material being carcinogenic. Related to biocompatibility is bioabsorbability, which may be an important factor in deciding the material to be used for a stationary tool and/or machining tool. A material that is bioabsorbable generally can be largely or entirely broken down inside the body. Biocompatible materials are not necessarily bioabsorbable, and it would be preferable for a stationary tool to be both biocompatible and bioabsorbable. Particularly in the application of this tool, it would be preferable if the material can be broken down quickly by the body, rather than over an extended period of time.

One concept tied to bioabsorbability is the dissolution rate of a material in a patient's body. The dissolution rate generally refers to a particular amount of material broken down over time. Another factor relevant to bioabsorbability of a material is the composition of the material. Specifically, materials that are native to a patient's body, such as magnesium, calcium, or carbon, may be more easily dealt with than non-native materials, such as rare earth metals. Generally, the body is already capable of processing, breaking down, eliminating, or otherwise dealing with native materials using normal mechanisms. This may be true even if such native materials are in the body in larger quantities than normal. On the other hand, the body may have a much more difficult time dealing with non-native materials, such as rare earth metals, that end up inside the body. This may be, in part, because the body has no particularly effective mechanism for responding to these non-native materials.

Other considerations may further be taken into account when choosing a material for use in a stationary tool and/or machining tool. For example, cost is almost always an important factor. This may refer to cost of the raw materials, and/or cost of processing those raw materials into a final product. Alloys for which such processes are already known, especially when those processes are efficient, may be more preferable from a cost standpoint. For example, an alloy that has known and/or efficient molding processes or that has a quick cycle time may be more preferable in terms of cost. Somewhat related to cost concerns is whether or not the material can be resterilized, for example by an autoclave. A material that may be resterilized can often be reused in later procedures, making cost somewhat less of a concern. Materials that cannot be resterilized generally cannot be used more than once in a surgical procedure, and generally must be used as disposable devices, which may make cost a larger concern.

For stationary tools that may be damaged by contact with a machining tool, it may be preferable that the stationary tool is used only a single time to eliminate the question of whether or not the stationary tool is structurally strong enough to withstand further use and/or contact with machining tools. For example, if a stationary tool is impacted by a machining tool during a first surgery, it may be weakened. If the stationary tool is resterilized and used for a second surgery, a decision may have to be made regarding whether or not the tool is capable of withstanding further contact or even simply withstanding further normal use. However, if a stationary tool is only to be used a single time, there is no decision to be made regarding whether the stationary tool should be resterilized and used in a later procedure. Eliminating the necessity for this decision may reduce the complexity of any particular surgical procedure. Further, the patient's safety is likely improved by eliminating the likelihood of reusing a weakened tool in a later procedure.

Materials traditionally used as stationary tools, such as retractors, are often composed of stainless steel or other tough, high modulus materials. These traditional stationary tools have the disadvantages of at least high toughness with little or no bioabsorbability, the disadvantages of which are described above. Certain bioabsorbable materials, such as poly-L-lactide, are composed of highly specialized polymers. These polymers, however, often have a modulus of elasticity and toughness that is too low, are reabsorbed relatively slowly, and are prohibitively expensive, the disadvantages of which are described above.

According to an embodiment of the invention, a magnesium alloy having at least one of a relatively high modulus of elasticity, low toughness relative to the machining tool, fast reabsorbtion characteristics and good biocompatibility is used to produce a stationary tool. The stationary tool formed with this magnesium alloy is capable of being inadvertently contacted during orthopedic procedures without imposing serious repercussions to the patient and hospital during and post-operatively.

Certain magnesium alloys have been used in the past for certain medical implants. For example, U.S. Patent Publication No. 2013/0041455 to Gerold ("Gerold") discusses the use of a bioabsorbable magnesium alloy for use in a stent. In one example, the magnesium alloy has a proportion of magnesium greater than 90% by weight, yttrium 3.7-5.5% by weight, rare earth metals 1.5-4.4% by weight, and the remainder less than 1% by weight. Gerold recognizes that although magnesium alloys have been used for implantable stents, screws, or plates, the alloy degrades in the body too quickly. Similarly, these magnesium alloys generally need to be close to the strength of bone to avoid stress shielding. There has been a push in the art, including by Gerold, to create magnesium alloys that have slower bioabsorbability characteristics. This would be useful, for example, so that a bone plate retains enough structural integrity to perform its function of splinting a fractured bone before the alloy is broken down. While magnesium alloys that have fast bioabsorbability characteristics may be unsuitable for many biomedical applications, the inventor has realized that they are particularly well suited for use in stationary tools in surgical systems.

Magnesium alloys may include one or more components besides magnesium. For example, secondary components may include alkali metals, alkaline earth metals, carbon, iron, zinc, manganese, cobalt, nickel, chromium, copper, cadmium, lead, tin, thorium, zirconium, silver, gold, palladium, platinum, silicon, calcium, lithium, and aluminum. Magnesium alloys may also include rare earth elements, including light rare earth elements (LRE; Lanthanum (La), Cerium (Ce), Praseodymium (Pr), Neodynium (Nd)) and heavy rare earth metals (HRE; Samarium (Sm), Europium (Eu), Gadolinium (Gd), Terbium (Tb), Dysprosium (Dy), Holmium (Ho), Erbium (Er), Thulium (Tm), Ytterbium (Yb) and Lutetium (Lu)). Generally, elements known to be toxic and/or carcinogenic to the body should be avoided when creating a magnesium alloy for biomedical applications. It may also be desirable to eliminate rare earth metals from the magnesium alloy. Among other reasons, rare earth metals are generally not native to the human body, and further, it may be difficult to determine exact compositions of rare earth metals in an alloy.

While the prior art suggests altering the composition of magnesium alloys so that they are slower to bioabsorb, magnesium alloys that are relatively quick to bioabsorb are particularly well suited for use in stationary tools in surgical systems. In fact, the stationary tools may benefit from the use of a magnesium alloy that is altered to bioabsorb in the body even faster. For example, dissolution rates of magnesium alloy may be altered by changing the amount of oxides in the alloy. Similarly, making the magnesium alloy more porous may decrease the amount of time for the alloy to bioabsorb in the body.

In one example, the magnesium alloy used in a stationary tool has a relatively low toughness and hardness compared to machining tools generally used in orthopedic procedures. The magnesium alloy still retains a sufficient magnitude of stiffness for applications such as retractors in which high elasticity is disfavored. For example, the strength may be approximately 150 MPa or greater. Unlike alloys to be used for bone plates, the strength of the alloy used for a stationary tool need not be limited to the general strength of the bone. In fact, the strength of the magnesium alloy for use in a stationary tool may be even greater than 150 MPa. The ductility of the magnesium alloy may be in the range of approximately 6% to approximately 8%.

The alloy generally cannot be resterilized by an autoclave. However, this is not a major issue because the alloy is relatively cheap and may be used for disposable stationary tools. The disposability is particularly desired for applications in which the stationary tool may be contacted by a machining tool, as there is little or no chance that a stationary tool weakened in a first procedure will break during a later second procedure using the stationary tool since the stationary tool is not to be used again. Of particular importance is the speed with which the magnesium alloy bioabsorbs in the body. In one illustrative embodiment, the magnesium alloy has a dissolution rate of between approximately 0.01 and approximately 0.12 grams per square centimeter per day. The dissolution rate of approximately 0.06 grams per square centimeter per day may be especially desired, as that rate will dissolve a cubic millimeter of a typical magnesium alloy (with a density of approximately 1.8 gram per cubic centimeter) in about 24 hours. For that same piece of alloy, the dissolution rate of approximately 0.01 grams per square centimeter per day would result in dissolution time of approximately 7 days, while a rate of approximately 0.12 would result in a dissolution rate of approximately 12 hours. Other ranges of dissolution rates within these ranges may also be preferred, for example between approximately 0.05 and approximately 0.07 grams per square centimeter per day. Extremely fast rates of dissolution may not be preferred, however, as these extreme rates may result in an exothermic reaction producing unwanted heat, and may also produce hydrogen gas. This production of hydrogen gas may result in hydrogen gas pockets in the body. For the particular applications described herein, however, hydrogen gas production may less of a concern than with larger implants, such as stents. With larger implants, the hydrogen gas pockets produced may be relatively large. However, the current disclosure is more likely to result in small chips or slivers of magnesium alloy in the body. High dissolution rates of these small pieces may result in hydrogen gas pockets, but these small pockets are likely to be dealt with by the body more easily than the larger hydrogen gas pockets that may be seen with large implants.

As described above, one particularly suitable use of this magnesium alloy is for retractors in orthopedic procedures in which a machining tool is also used. Although described in the context of a knee surgery using a limb holder, retractors used in any orthopedic surgeries with a machining tool could benefit from being formed of the magnesium alloy described above. However, the invention is not limited to retractors. Nearly any procedure in which a stationary tool is used along with a machining tool would benefit from the stationary tool being formed of the magnesium alloy described above. For example, in certain total knee arthroplasty procedures, one or more guides with cutting slots or cutting guide surfaces may be used to guide a reciprocating saw along the surface during resection of the femur or tibia.

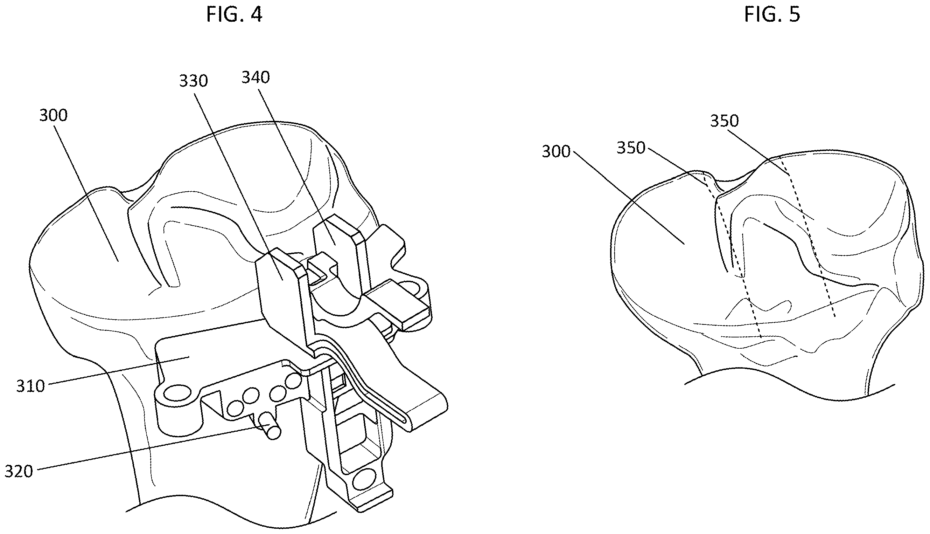

For example, FIG. 4 illustrates proximal tibia 300 being prepared for resection for later implantation of a tibial baseplate of a prosthetic knee. One particular resection cutting guide 310 that may be used in the resection is illustrated being coupled to the proximal tibia 300 by virtue of a pin 320. Also attached to the resection cutting guide 310 is a vertical cutting guide including guide walls 330 and 340. This system and other similar systems are described more fully in Collazo et al., U.S. Patent Application No. 61/767,954, titled "Bicruciate Retaining Tibial Implant System" and filed on Feb. 22, 2013.

FIG. 5 illustrates the proximal tibia 300 after sagittal cuts 350 have been made in the tibia. To perform these cuts, a surgeon uses a reciprocating saw or other machining tool. The surgeon glides the saw blade along the outside of the guide walls 330 and 340 to make the two sagittal cuts 350. As the cut is made, the saw blade is likely to make contact with one or both of the guide walls 330, 340. As the cutting continues, the surgeon may also contact the saw blade with a top surface of the resection cutting guide 310. Even though such contact may be intentional, the contact may deform the guide walls 330, 340 and/or the resection guide 310, especially if more force is used than intended by the surgeon.

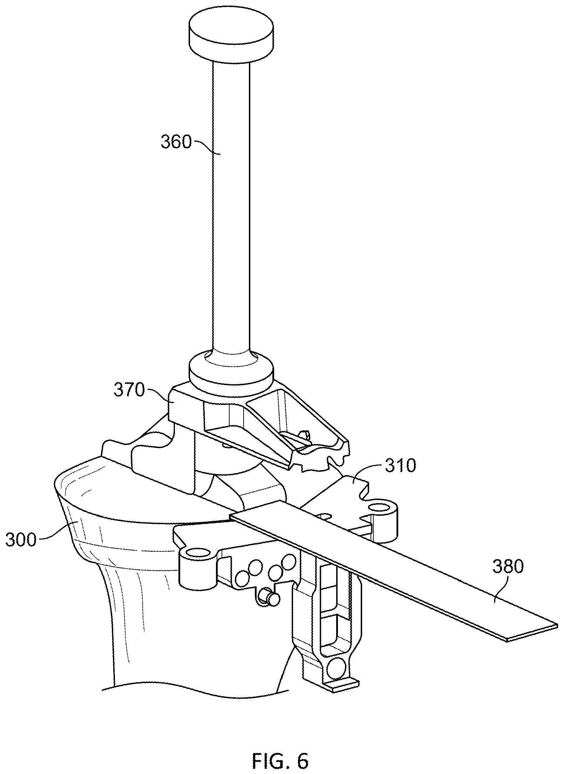

FIG. 6 illustrates an additional step in the resection procedure, after a punch 360 has been inserted into the tibial eminence and after a tibial eminence protector 370 has been inserted into the sagittal cuts 350 described above. After the punch has been made with the punch 360 and the tibial eminence protector 370 is in place, the surgeon makes a resection cut on the proximal tibia 300 with a reciprocating saw 380 (or other machining tool). The surgeon may use the top surface of the resection cutting guide 310 to guide this cut. The surgeon may move the saw blade 380 until it makes contact with the tibial eminence protector 370.

As can be seen, there are multiple opportunities for the saw blade 380 to make contact with stationary components such as the resection cutting guide 310 or the tibial eminence protector 370. While such contact may be intended, it is possible that the saw blade can be dulled from such contact, and that pieces of the stationary tools, however small, will break off and end up in the patient's body. As described above with regard to the retractors, one or both of the stationary tools (in this case, resection cutting guide 310 and tibial eminence protector 370) could be formed of the above described magnesium alloy. Again, the magnesium alloy would benefit the surgical system in the same ways as described above in relation to the retractor and limb holder system.

It should further be recognized that the same or similar benefit may be obtained from using a stationary tool that is only partially made of the described magnesium alloy. For example, referring back to retractors 202 and 202' of FIGS. 2-3, the same benefits of using a magnesium alloy to form the entire retractor may be obtained from using a magnesium alloy coating on top of a more traditional material. With a magnesium alloy coating, most or all of the material chipped off the stationary tool from contact with a machining tool would be the biocompatible and bioabsorbable magnesium alloy. Similarly, the magnesium alloy coating would have similar or the same hardness and toughness properties as if the entire stationary tool was formed of magnesium alloy.

Still further, the same or similar benefits may be obtained from using an insert of magnesium alloy with a stationary tool formed of a more traditional material such as stainless steel. For example, retractor 202 may be entirely made of stainless steel. A magnesium alloy insert into which the retractor 202 can be inserted may be used in conjunction with the retractor. By using a magnesium alloy insert, the surface presented to the machining tool and the surgical site largely or entirely has the advantageous properties of the magnesium alloy described. A further benefit from using such an insert is that the insert itself may be disposable, while the remaining portion of the stationary tool that is unlikely to make contact with a machining tool is formed of a reusable, resterilizable traditional material. Such an insert would preferably cover at least a surface of the stationary tool subject to likely impact with, or wear by, a machining tool such as drill bit.

Similar magnesium alloy inserts could be used with a variety of stationary tools. For example, the guide walls 330, 340 of the vertical cutting guide in FIG. 4 could each be provided with an insert which slips over the respective guide walls. Similarly, an insert with a similar shape as the top surface of the resection cutting guide 310 may be temporarily attached to the resection cutting guide for use during a surgical procedure. Once the procedure is complete, the insert(s) may be removed and discarded, with the stationary components discarded or resterilized for use in further procedures.

A further application of a stationary tool with magnesium alloy is for fixtures for guiding drills when drilling into a bone. Often, prior to drilling into bone, a smaller pilot hole is made in the bone to guide the drill. When using a fixture to guide the drill, for example during drilling a pilot hole, a fixture or other guide with an aperture is used. The size of the aperture is generally larger than the size of the particular drill, such that the drill may be passed through the fixture without making contact with the walls of the fixture, which may be made of a hard material, such as stainless steel. By forming the fixture at least partially from magnesium alloy, the guide aperture may be smaller than traditional guide apertures, and even smaller than the particular drill bit itself. This would allow for essentially zero clearance, providing highly accurate drill guiding. Although the drill bit would make contact with the walls of the guide aperture, the shavings created are bioabsorbable. These shavings entering the body of the patient may be broken down by the body. Further, the drill bit would not become as deformed as one would see if using a more traditional material, such as stainless steel, for the drill guide.

Although not illustrated, there are numerous other orthopedic surgical systems that include a machining tool and a stationary tool that would benefit from the stationary tool being partially or entirely composed of the magnesium alloy described above, partially or entirely coated with magnesium alloy, or used in conjunction with magnesium alloy inserts. For example, distal resection guides for the femur, as well as 4-in-1 cutting blocks used after the distal resection to create anterior, anterior chamfer, posterior, and posterior chamfer cuts, could be composed of magnesium alloy to reap the benefits described above. Alternately, guides in the distal resection guide or 4-in-1 cutting block, such as slots or grooves for reciprocating saws, may be coated with magnesium alloy or provided with a magnesium alloy insert, largely as described above with reference to FIGS. 4-7.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the present invention as defined by the appended claims.

## Claims

1. A surgical system for performing a surgical procedure comprising:
a machining tool having a first contact surface having a first hardness; and
a stationary tool (202, 202', 310) having a second contact surface at least partially formed of a magnesium alloy, the second contact surface being biocompatible and bioabsorbable and having a second hardness,
**characterized in that** the first hardness is greater than the second hardness, and **in that** the second contact surface of the stationary tool includes a magnesium alloy coating.

2. The surgical system of claim 1, wherein the first contact surface has a toughness that is greater than a toughness of the second contact surface.

3. The surgical system of claim 1, wherein the machining tool is selected from the group consisting of a bur, a drill bit, an end mill, and a saw.

4. The surgical system of claim 1, wherein the stationary tool is selected from the group consisting of a retractor (202, 202'), a jig, a fixture, and a cutting guide (310).

5. The surgical system of claim 1, wherein the magnesium alloy has a dissolution rate of between 0.01 grams per square centimeter per day and 0.12 grams per square centimeter per day.

6. The surgical system of claim 5, wherein the magnesium alloy has a dissolution rate of between 0.05 grams per square centimeter per day and 0.07 grams per square centimeter per day.

## Patentansprüche

1. Chirurgisches System zum Durchführen eines chirurgischen Eingriffs, umfassend:
ein Bearbeitungswerkzeug, aufweisend eine erste Kontaktfläche mit einer ersten Härte; und
ein stationäres Werkzeug (202, 202', 310), aufweisend eine zweite Kontaktfläche, die wenigstens teilweise aus einer Magnesiumlegierung gebildet ist, wobei die zweite Kontaktfläche biokompatibel und bioabsorbierbar ist und eine zweite Härte aufweist,
**dadurch gekennzeichnet, dass** die erste Härte größer als die zweite Härte ist, und dass die zweite Kontaktfläche des stationären Werkzeugs eine Beschichtung aus einer Magnesiumlegierung aufweist.

2. Chirurgisches System nach Anspruch 1, wobei die erste Kontaktfläche eine Zähigkeit aufweist, die höher als eine Zähigkeit der zweiten Kontaktfläche ist.

3. Chirurgisches System nach Anspruch 1, wobei das Bearbeitungswerkzeug ausgewählt ist aus der Gruppe bestehend aus einem Fräser, einem Bohreinsatz, einem Schaftfräser und einer Säge.

4. Chirurgisches System nach Anspruch 1, wobei das stationäre Werkzeug ausgewählt ist aus der Gruppe bestehend aus einem Retraktor (202, 202'), einer Schablone, einer Spannvorrichtung und einer Schnittführung (310).

5. Chirurgisches System nach Anspruch 1, wobei die Magnesiumlegierung eine Auflösungsgeschwindigkeit von zwischen etwa 0,01 Gramm pro Quadratzentimeter pro Tag und etwa 0,12 Gramm pro Quadratzentimeter pro Tag aufweist.

6. Chirurgisches System nach Anspruch 5, wobei die Magnesiumlegierung eine Auflösungsgeschwindigkeit von zwischen etwa 0,05 Gramm pro Quadratzentimeter pro Tag und etwa 0,07 Gramm pro Quadratzentimeter pro Tag aufweist.

## Revendications

1. Système chirurgical pour réaliser une procédure chirurgicale comprenant :
un outil d'usinage ayant une première surface de contact ayant une première dureté ; et
un outil fixe (202, 202', 310) ayant une seconde surface de contact au moins partiellement formée avec un alliage de magnésium, la seconde surface de contact étant biocompatible et bioabsorbable et ayant une seconde dureté,
**caractérisé en ce que** la première dureté est supérieure à la seconde dureté, et **en ce que** la seconde surface de contact de l'outil fixe comprend un revêtement en alliage de magnésium.

2. Système chirurgical selon la revendication 1, dans lequel la première surface de contact a une ténacité qui est supérieure à une ténacité de la seconde surface de contact.

3. Système chirurgical selon la revendication 1, dans lequel l'outil d'usinage est sélectionné dans le groupe constitué d'une fraise, un foret, une fraise cylindrique et une scie.

4. Système chirurgical selon la revendication 1, dans lequel l'outil fixe est sélectionné dans le groupe constitué d'un rétracteur (202, 202'), un gabarit, un dispositif de fixation et un guide de coupe (310).

5. Système chirurgical selon la revendication 1, dans lequel l'alliage de magnésium a une vitesse de dissolution comprise entre 0,01 gramme par centimètre carré par jour et 0,12 gramme par centimètre carré par jour.

6. Système chirurgical selon la revendication 5, dans lequel l'alliage de magnésium a une vitesse de dissolution comprise entre 0,05 gramme par centimètre carré par jour et 0,07 gramme par centimètre carré par jour.
